# EUROPÄISCHE PATENTSCHRIFT

(11) **EP 0 900 240 B1**
(45) Veröffentlichungstag und Bekanntmachung des Hinweises auf die Patenterteilung: **03.12.2003**
(21) Anmeldenummer: 97923951.4
(22) Anmeldetag: 20.05.1997
(51) Int. Cl.: C08F 8/00

(54) **VERFAHREN ZUR HERSTELLUNG VON POLYALKENAMINEN**
PROCESS FOR PREPARING POLYALKENE AMINES
PROCEDE DE PREPARATION D'AMINES DE POLYALKENE

(30) Priorität: 20.05.1996 DE 19620262
(43) Veröffentlichungstag der Anmeldung: 10.03.1999
(73) Patentinhaber: BASF AKTIENGESELLSCHAFT, 67056 Ludwigshafen (DE)
(72) Erfinder: MELDER, Johann-Peter, D-67141 Neuhofen (DE); BLUM, Gerhard, D-67117 Limburgerhof (DE); GÜNTHER, Wolfgang, D-67582 Mettenheim (DE); POSSELT, Dietmar, D-69120 Heidelberg (DE); OPPENLÄNDER, Knut, D-67061 Ludwigshafen (DE)
(74) Vertreter: Kinzebach, Werner, Dr.
(86) Internationale Anmeldenummer: EP9702571
(87) Internationale Veröffentlichungsnummer: WO97044366

(56) Entgegenhaltungen:
- EP-A- 0 382 405
- EP-A- 0 385 039
- EP-A- 0 561 214
- EP-A- 0 617 056
- WO-A-92/14806

## Beschreibung

Die vorliegende Erfindung betrifft ein Verfahren zur Herstellung von Polyalkenaminen aus Epoxiden. Die erfindungsgemäß hergestellten Produkte finden insbesondere Verwendung als Kraft- und Schmierstoffadditive.

Vergaser und Einlaßsysteme von Ottomotoren, aber auch Einspritzsysteme für die Kraftstoffdosierung in Otto- und Dieselmotoren werden in zunehmendem Maße durch Verunreinigungen belastet. Die Verunreinigungen werden verursacht durch Staubteilchen aus der vom Motor angesaugten Luft, unverbrannte Kohlenwasserstoffreste aus dem Brennraum und die in den Vergaser geleiteten Entlüftungsgase aus dem Kurbelwellengehäuse.

Diese Rückstände verschieben das Luft-Kraftstoffverhältnis im Leerlauf und im unteren Teillastbereich, so daß das Gemisch fetter und die Verbrennung unvollständiger wird. Als Folge davon erhöht sich der Anteil unverbrannter oder teilverbrannter Kohlenwasserstoffe im Abgas und der Benzinverbrauch steigt.

Es ist bekannt, daß zur Vermeidung dieser Nachteile Kraftstoffadditive zur Reinhaltung von Ventilen und Vergaser- bzw. Einspritzsystemen verwendet werden (vgl. z.B.: M. Rossenbeck in Katalysatoren, Tenside, Mineralöladditive, Hrsg. J. Falbe, U. Hasserodt, S. 223, G. Thieme Verlag, Stuttgart 1978). Je nach Wirkungsweise und bevorzugtem Wirkort solcher Detergens-Additive unterscheidet man heute zwei Generationen. Die erste Additiv-Generation konnte nur die Bildung von Ablagerungen im Ansaugsystem verhindern, nicht aber bereits vorhandene Ablagerungen wieder entfernen. Die Additive der zweiten Generation können dagegen Ablagerungen verhindern und beseitigen (keep-clean- und clean-up-Effekt). Dies wird insbesondere durch deren hervorragende Thermostabilität an Zonen höherer Temperatur, wie insbesondere an den Einlaßventilen, ermöglicht.

Das molekulare Bauprinzip dieser als Detergenzien wirkenden Additive der zweiten Generation beruht auf der Verknüpfung polarer Strukturen mit meist höhermolekularen, unpolaren oder oleophilen Resten. Typische Vertreter der zweiten Additiv-Generation sind Produkte auf der Basis von Polyisobuten im unpolaren Molekülteil, wie insbesondere Additive vom Polyisobutenamin-Typ und vom Polyisobutenaminoalkohol-Typ. Derartige Detergenzien sind, ausgehend von Polyisobutenen, nach verschiedenen mehrstufigen Syntheseverfahren herstellbar.

Polyisobutenaminoalkohole werden hergestellt, indem man Polyisobutene zunächst epoxidiert und das Epoxid anschliessend mit dem gewünschten Amin umsetzt. Derartige homogen oder heterogen katalysierte Verfahren sind z.B. beschrieben in der WO 92/12221, der WO 92/14806, der EP 0 476 485 sowie der EP 0 539 821.

Polyisobutenamine erhält man, ausgehend von Polyisobuten, im wesentlichen nach zwei Verfahren.

Das erste Verfahren verläuft über eine Chlorierung des polymeren Grundkörpers, gefolgt von einer nukleophilen Substitution durch Amine oder bevorzugt Ammoniak. Nachteilig bei diesem Verfahren ist die Verwendung von Chlor, die zur Folge hat, daß chlor- oder chloridhaltige Produkte auftreten, was heute keinesfalls mehr erwünscht ist, und wenn möglich, vermieden werden soll. So beschreiben beispielsweise die DE-OS 21 29 461 und die DE-OS 22 45 918 die Umsetzung halogenhaltiger Kohlenwasserstoffe mit einer Aminverbindung in Gegenwart eines Halogenwasserstoff-Akzeptors.

Im zweiten Verfahren werden die Polyisobutenamine ausgehend von Polyisobuten über Hydroformylierung und anschliessende reduktive Aminierung hergestellt. So beschreiben bespielsweise die EP 0 244 616 und die DE-PS 36 11 230 die Carbonylierung von Polybuten oder Polyisobuten in Gegenwart eines homogenen Katalysators, wie z.B. Kobaltoctacarbonyl, und die anschliessende Umsetzung des Oxoproduktes zum Amin. Nachteilig bei diesem Verfahren sind der hohe technische Aufwand zur Carbonylierung des reaktiven Polyisobutens unter Hochdruckbedingungen sowie die speziellen Maßnahmen zur Entfernung des homogenen Carbonylierungskatalysators.

Der Erfindung liegt somit die Aufgabe zugrunde, ein Verfahren zur Herstellung von Polyalkenaminen bereitzustellen, das im Vergleich zu den bisher bekannten Verfahren einfacher durchzuführen ist und ein im wesentlichen halogenidfreies Produkt liefert. Insbesondere soll das neuartige Verfahren, ausgehend vom Polyalken, ohne die aufwendige Oxosynthese durchgeführt werden können.

Es wurde nun gefunden, daß diese Aufgabe gelöst wird durch Bereitstellung eines Verfahrens zur Herstellung von Polyalkenaminen der Formel (I) 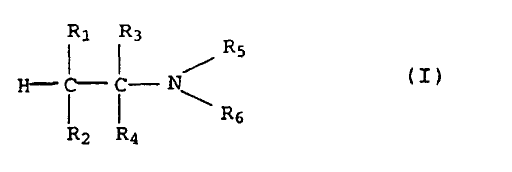 worin
- R₁, R₂, R₃ und R₄: unabhängig voneinander für ein Wasserstoffatom oder einen gegebenenfalls substituierten, gesättigten oder ein- oder mehrfach ungesättigten aliphatischen Rest mit einem zahlenmittleren Molekulargewicht von bis zu etwa 40000 steht; wobei wenigstens einer der Reste R₁ bis R₄ ein zahlenmittleres Molekulargewicht von etwa 150 bis etwa 40000 aufweist; und
- R₅ und R₆: unabhängig voneinander für ein Wasserstoffatom, einen Alkyl-, Cycloalkyl-, Hydroxyalkyl-, Aminoalkyl-, Alkenyl-, Alkinyl-, Aryl-, Arylalkyl-, Alkylaryl-, Heteroarylrest oder einen Alkyleniminrest der Formel (II) 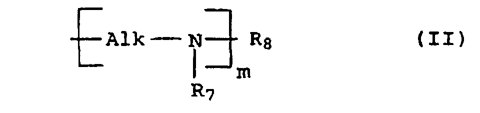 stehen, worin
Alk für einen geradkettigen oder verzweigten Alkylenrest steht;
m für einen ganzzahligen Wert von 0 bis 10 steht, und
R₇ und R₈ unabhängig voneinander für ein Wasserstoffatom, einen Alkyl-, Cycloalkyl-, Hydroxyalkyl-, Aminoalkyl-, Alkenyl-, Alkinyl-, Aryl-, Arylalkyl-, Alkylaryl- oder Heteroarylrest stehen oder zusammen mit dem Stickstoffatom, an das sie gebunden sind, einen Heterozyklus bilden; oder
R₅ und R₆ zusammen mit dem Stickstoffatom, an das sie gebunden sind, für einen Heterozyklus stehen; wobei jeder der Reste R₅, R₆, R₇ und R₈ durch weitere Hydroxy- oder Aminogruppen tragende Alkylreste substituiert sein kann;
wobei man ein Epoxid der Formel (IV) 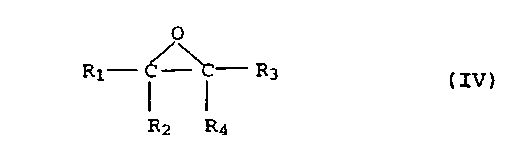 worin R₁, R₂, R₃ und R₄ die oben angegebenen Bedeutungen besitzen, mit einer Stickstoffverbindung der Formel (V) 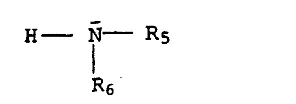 worin R₅ und R₆ die oben angegebenen Bedeutungen besitzen, zum Aminoalkohol der Formel (VI) 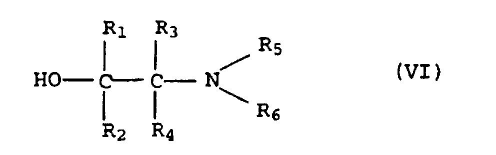 umsetzt;
den Aminoalkohol der Formel (VI) katalytisch dehydratisiert, und
das gebildete Olefin zum Amin der Formel (I) hydriert.

Gemäß einer ersten bevorzugten Ausführungsform erfolgt die Umsetzung des Epoxids (IV) zum Amin (I) einstufig, indem man das Epoxid (IV) mit der Stickstoffverbindung (V) in Gegenwart von Wasserstoff und von einem Katalysator umsetzt, welcher Dehydratisierungs- und zugleich Hydrierungseigenschaften besitzt.

Gemäß einer zweiten bevorzugten Ausführungsform erfolgt die Umsetzung des Epoxids (IV) zum Amin (I) in zwei Stufen, indem man das Epoxid (IV) zuerst mit der Stickstoffverbindung (V) in Gegenwart eines Alkoxylierungskatalysators zum Aminoalkohol (VI) umsetzt und gegebenenfalls nicht umgesetzte Reaktanden abtrennt. Der Aminoalkohol (VI) wird in einer zweiten Stufe in Gegenwart eines Katalysators, welcher Dehydratisierungs- und zugleich Hydrierungseigenschaften besitzt, zum Amin (I) hydriert.

Die zweite Verfahrensvariante ist insbesondere dann zweckmäßig, wenn Reaktanden eingesetzt werden, die unter den gewählten Reaktionsbedingungen unerwünschte Nebenreaktionen eingehen können. Dies kann z.B. der Fall sein bei Verwendung von Ethylendiamin als Stickstoffverbindung der Formel (V). In Gegenwart der erfindungsgemäß verwendeten Katalysatoren mit Dehydratisierungs- und Hydrierungseigenschaften kann es hierbei zur Dimerisierung unter Bildung von Piperazin kommen, was vermieden werden kann, wenn man in einer ersten Verfahrensstufe zunächst den Aminoalkohol (VI) erzeugt, nicht umgesetztes Amin entfernt, und anschliessend nach Katalysatorzugabe zum Endprodukt (I) dehydratisiert und hydriert.

Der erfindungsgemäß verwendbare Katalysator mit Dehydratigierungs- und Hydrierungseigenschaften ist vorzugsweise ausgewählt unter Zeolithen oder porösen Oxiden von Al, Si, Ti, Zr, Nb, Mg und/oder Zn, sauren Ionenaustauschern und Heteropolysäuren, welche jeweils mindestens ein Hydriermetall tragen. Als Hydriermetall verwendet man bevorzugt Ni, Co, Cu, Fe, Pd, Pt, Ru, Rh oder Kombinationen davon.

Erfindungsgemäß brauchbare Zeolithe sind beispielsweise saure zeolithische Feststoffkatalysatoren, die beschrieben sind in der EP 0 539 821, worauf hiermit Bezug genommen wird. Als Beispiele für geeignete Zeolithe sind zu nennen Zeolithe mit Mordenit-, Chabasit- oder Faujasit-Struktur; Zeolithe vom Typ A, L, X und Y; Zeolithe vom Pentasiltyp mit MFI-Struktur; Zeolithe, in denen Aluminium und/oder Silizium ganz oder teilweise durch Fremdatome ersetzt sind, wie z.B. Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische oder Titansilikatzeolithe, wie TS-1, ETS 4 und ETS 10.

Zur Optimierung von Selektivität, Umsatz und Standzeiten können die erfindungsgemäß verwendeten Zeolithe in geeigneter Weise mit weiteren Elementen dotiert werden, wie dies z.B. in der EP 0 539 821 beschrieben ist.

In gleicher Weise kann eine Dotierung der Zeolithe mit obengenannten Hydriermetallen erfolgen. Das Hydriermetall sollte, berechnet als Oxid, in einem Anteil von 1 bis 10 Gew.-%, bezogen auf das Gesamtgewicht der katalytisch aktiven Masse, enthalten sein.

Weitere geeignete Katalysatoren mit Dehydratisierungs- und Hydrierungseigenschaften sind, vorzugsweise saure, Oxide der Elemente Al, Si, Zr, Nb, Mg oder Zn oder Gemische davon, die mit wenigstens einem der obengenannten Hydriermetalle dotiert sind. Das Oxid (berechnet als Al₂O₃, SiO₂, ZrO₂, Nb₂O₅, MgO oder ZnO) ist dabei in einem Anteil von etwa 10 bis 99 Gew.-%, vorzugsweise etwa 40 bis 70 Gew.-%, in der Katalysatormasse (d.h. katalytisch aktiven Masse) enthalten. Das Hydriermetall (berechnet als NiO, CoO, CuO, Fe₂O₃, PdO, PtO, RuO₂ oder Rh₂O₃) ist dabei in einem Anteil von etwa 1 bis 90 Gew.-%, vorzugsweise etwa 30 bis 60 Gew.-%, bezogen auf das Gesamtgewicht der Katalysatormasse, enthalten. Außerdem können in den erfindungsgemäß eingesetzten Oxiden geringe Mengen, d.h. etwa 0,1 bis etwa 5 Gew.-% (berechnet für die Oxide) weiterer Elemente, wie z.B. Mo oder Na enthalten sein, um Katalysatoreigenschaften, wie z.B. Selektivität und Standzeit zu verbessern.

Oxide dieses Typs und deren Herstellung sind beispielsweise beschrieben in der EP 0 696 572, worauf hiermit Bezug genommen wird. Die Herstellung erfolgt vorzugsweise dadurch, daß man eine wässrige Salzlösung herstellt, welche die obengenannten Katalysator-Komponenten enthält, und durch Zugabe einer Mineralbase, wie z.B. Natriumcarbonat, gegebenenfalls unter leichtem Erwärmen, copräzipitiert. Den Niederschlag trennt man ab, wäscht, trocknet und calziniert, wie z.B. durch 4-stündiges Erwärmen auf 500°C.

Die obenbeschriebenen erfindungsgemäßen Zeolithe und aktiven Oxide können gegebenenfalls konditioniert werden, indem man sie gegebenenfalls auf eine bestimmte Korngröße vermahlt und zu Strängen oder Tabletten presst, wobei Formhilfsmittel, wie z.B. Graphit, zugesetzt werden können.

Erfindungsgemäß besonders bevorzugt ist die Verwendung eines Katalysators, der, bezogen auf das Gesamtgewicht der katalytisch aktiven Masse,
etwa 30 Gew.-% Zr, berechnet als ZrO₂,
etwa 50 Gew.-% Ni, berechnet als NiO,
etwa 18 Gew.-% Cu, berechnet als CuO,
etwa 1,5 Gew.-% Mo, berechnet als MoO₃ und
etwa 0,5 Gew.-% Na, berechnet als Na₂O enthält.

Alkoxylierungskatalysatoren, welche dem Reaktionsgemisch erfindungsgemäß vorzugsweise zugesetzt werden, fördern die Öffnung des Epoxid-Ringes. Beispiele für geeignete Alkoxylierungskatalysatoren sind Wasser und Alkohole, wie Methanol, Ethanol, Mineralsäuren und Carbonsäuren.

Das als Ausgangsprodukt zur Herstellung des Epoxids der Formel (IV) eingesetzte Polyalken der Formel (III) 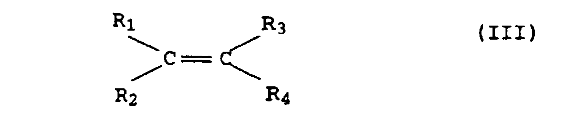 ist ein von mindestens einem geradkettigen oder verzweigten C₂-C₃₀-Alken, vorzugsweise C₂-C₆-Alken, insbesondere C₂-C₄-Alken abgeleitetes Polymer, wobei mindestens einer der Reste R₁ bis R₄ ein zahlenmittleres Molekulargewicht von etwa 150 bis 40000 aufweist.

Als Beispiele für C₂-C₄-Alkene sind zu nennen Ethylen, Propylen sowie insbesondere 1-Buten und Isobuten.

Die erfindungsgemäß bevorzugt verwendeten Polyalkene der Formel (III) sind reaktive Polyalkene mit einem hohen Anteil an endständigen Doppelbindungen. Eine Möglichkeit zur Herstellung reaktiver Polyalkene ist beispielsweise in der DE-OS 27 02 604 beschrieben.

Besonders bevorzugt ist Polyisobuten mit einem zahlenmittleren Molekulargewicht von etwa 800 bis 1500.

Außerdem sind erfindungsgemäß einsetzbar reaktive Polypropylene. Diese erhält man insbesondere durch Metallocen-Katalyse gemäß DE-OS 42 05 932 und weisen endständige Doppelbindungen auf, die überwiegend als Vinylidengruppe vorliegen. Vinylterminierte Polypropylene erhält man beispielsweise gemäß EP 0 268 214.

Auf die Offenbarung der obengenannten Patentanmeldungen wird hiermit ausdrücklich Bezug genommen.

Bevorzugte Katalysatorsysteme zur Herstellung vinylterminierter Polymere sind Bis(pentamethyl-cyclopentadienyl)zirkoniumdichlorid sowie Bis(pentamethyl-cyclopentadienyl)hafniumdichlorid in toluolischer Methylalumoxanlösung.

Bevorzugte Katalysatoren zur Herstellung vinylidenterminierter Polymere sind Bis(n-butylcyclopentadienyl)zirkoniumdichlorid, Bis(octadecylcyclopentadienyl)zirkoniumdichlorid und Bis(tetrahydroindenyl)zirkoniumdichlorid jeweils in toluolischer Methylalumoxanlösung.

Die obenbeschriebenen Polyalkene der Formel (III) werden zunächst in das Epoxid der Formel (IV) überführt. Die Epoxidation wird beispielsweise so durchgeführt, daß man das Polyalken in einem geeigneten Lösungsmittel, wie z.B. Diethylether oder anderen dipolar aprotischen Lösungsmitteln oder apolaren Lösungsmitteln, wie Xylol oder Toluol, löst, diese Lösung gegebenenfalls trocknet, das Epoxidationsmittel zusetzt und gegebenenfalls unter leichtem Erwärmen, z.B. auf etwa 40 bis 70°C, epoxidiert. Zur Durchführung der Epoxidation verwendet man herkömmliche Epoxidationsmittel. Beispiele hierfür sind Persäuren, wie Peroxybenzoesäure, m-Chlorperoxybenzoesäure oder Peroxyessigsäure oder Alkylperoxide, wie tert.-Butylhydroperoxid, wobei m-Chlorperbenzoesäure und Peroxyessigsäure bevorzugt sind.

Bei der Epoxidation können Epoxide unterschiedlicher stereoisomerer Form einzeln oder im Gemisch ausfallen, wie z.B. Verbindungen der allgemeinen Formeln (IVa), (IVb), (IVc) und (IVd)

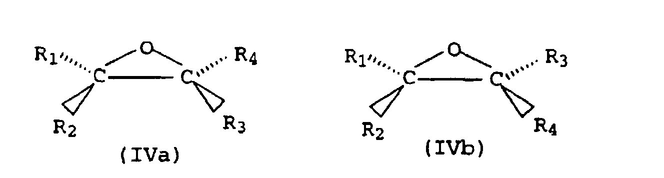

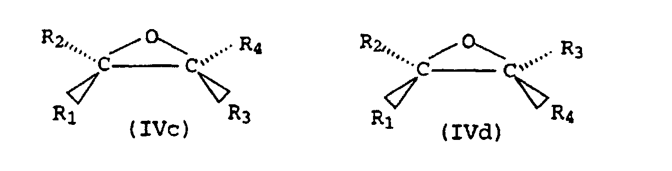

Zur Umsetzung mit der Stickstoffverbindung der Formel (V) kann man ein bestimmtes Isomer einsetzen; gewöhnlich verwendet man aber ein Isomerengemisch zur Durchführung der Aminierung.

Beispiele für geeignete Stickstoffverbindungen der Formel (V) sind Ammoniak, Ethylen-1,2-diamin, Propylen-1,2-diamin, Propylen-1,3-diamin, Butylendiamine, die Monoalkyl-, Dialkyl- und Trialkylderivate dieser Amine, wie z.B. N,N-Dimethylpropylen-1,3-diamin. Ferner können Polyalkylenpolyamine eingesetzt werden, deren Alkylenreste nicht mehr als 6 C-Atome aufweisen, wie z.B. Polyethylenpolyamine, wie Diethylentriamin, Triethylentetramin und Tetraethylenpentamin, und Polypropylenpolyamine. Weitere Beispiele sind N-Amino-C₁-C₆-alkylpiperazine. Bevorzugt verwendet man Ammoniak.

Die Epoxide werden in beiden der obenbeschriebenen Verfahrensvarianten, die sowohl kontinuierlich als auch diskontinierlich durchgeführt werden können, bei Temperaturen von etwa 80 bis 250°C, vorzugsweise etwa 150 bis 210°C und Wasserstoffdrücken bis etwa 600 bar, vorzugsweise etwa 80 bis 300 bar mit der Stickstoffverbindung der Formel (V) umgesetzt. Die Stickstoffverbindung wird, bezogen auf das Epoxid, in einem molaren Verhältnis von etwa 1:1 bis etwa 40:1, bevorzugt in einem Überschuß von etwa 5:1 bis etwa 20:1, eingesetzt. Die Umsetzung kann sowohl in Substanz als auch in Gegenwart eines Lösungsmittels (z.B. Kohlenwasserstoffe, wie etwa Hexan, oder THF) durchgeführt werden.

Die in den erfindungsgemäß hergestellten Verbindungen der Formel (I) enthaltenen Alkylreste umfassen insbesondere geradkettige oder verzweigte, gesättigte Kohlenstoffketten mit 1 bis 10 Kohlenstoffatomen. Beispielsweise können folgende Reste genannt werden: Niedrigalkylreste, d.h. C₁-C₆-Alkylreste, wie Methyl, Ethyl, n-Propyl, i-Propyl, n-Butyl, sec.-Butyl, i-Butyl, t-Butyl, n-Pentyl, sec.-Pentyl, i-Pentyl, n-Hexyl, 1-, 2- oder 3-Methylpentyl; längerkettige Alkylreste, wie unverzweigtes Heptyl, Octyl, Nonyl und Decyl und die verzweigten Analoga davon.

Die erfindungsgemäß hergestellten Verbindungen können gegebenenfalls Hydroxy- und Aminoalkylreste enthalten, worin der Alkylteil wie oben definiert ist und sich die Hydroxy- oder Aminogruppe vorzugsweise an einem terminalen Kohlenstoffatom befindet.

Die in den erfindungsgemäß hergestellten Verbindungen enthaltenen Alkenylreste umfassen insbesondere geradkettige oder verzweigte Kohlenstoffketten mit wenigstens einer Kohlenstoff-Kohlenstoff-Doppelbindung und mit 2 bis 10 Kohlenstoffatomen. Als Beispiele für einfach ungesättigte C₂-C₁₀-Alkenylreste können genannt werden: Vinyl, Allyl, 1-Propenyl, Isopropenyl, 1-, 2- oder 3-Butenyl, Methallyl, 1,1-Dimethylallyl, 1-, 2-, 3-, 4- oder 5-Hexenyl; längerkettige Reste, wie unverzweigtes Heptenyl, Octenyl, Nonenyl und Decenyl und die verzweigten Analoga davon, wobei die Doppelbindung in beliebiger Position auftreten kann. Erfindungsgemäß mitumfasst werden sowohl die cis- als auch die trans-Isomeren obiger C₂-C₁₀-Alkenylreste.

Die in den erfindungsgemäß hergestellten Verbindungen enthaltenen Alkinylreste umfassen insbesondere geradkettige oder verzweigte Kohlenstoffketten mit wenigstens einer Kohlenstoff-Kohlenstoff-Dreifachbindung und 2 bis 10 Kohlenstoffatomen. Beispiele umfassen Ethinyl, 1- oder 2-Propinyl, 1-, 2- oder 3-Butinyl sowie die entsprechenden Alkinylanaloga oben genannter Alkenylreste.

Beispiele für erfindungsgemäß verwendbare Cycloalkylgruppen umfassen insbesondere C₃-C₇-Cycloalkylreste, wie Cyclopropyl, Cyclobutyl, Cyclopentyl, Cyclohexyl, Cycloheptyl, Cyclopropylmethyl, Cyclopropylethyl, Cyclopropylpropyl, Cyclobutylmethyl, Cyclobutylethyl, Cyclopentylethyl und dergleichen.

Beispiele für erfindungsgemäß verwendbare Arylreste sind Phenyl und Naphthyl.

Erfindungsgemäß verwendbarer Arylalkylreste sind insbesondere Phenyl-C₁-C₁₀-alkyl und Naphthyl-C₁-C₁₀-alkyl, und Beispiele für geeignete Alkylarylreste sind C₁-C₁₀-Alkyl-phenyl und C₁-C₁₀-Alkylnaphthyl, wobei jeweils der C₁-C₁₀-Alkylteil wie oben definiert ist.

Die in den erfindungsgemäß hergestellten Verbindungen enthaltenen Cycloalkyl-, Aryl- und Arylalkylgruppen können gegebenenfalls 1 oder mehrere, wie z.B. 1 bis 4, Heteroatome, wie O, S und N enthalten, wobei Sauerstoff und Stickstoff als Heteroatom bevorzugt sind. Beispiele für cyclische Heteroalkylreste sind Tetrahydrofuranyl, Piperidinyl, Piperazinyl und Morpholinyl. Beispiele für Heteroarylgruppen sind 5- oder 6-gliedrige aromatische Ringsysteme, die 1 bis 4 der genannten Heteroatome umfassen, wie z.B. Furyl, Pyrrolyl, Imidazolyl, Pyrazolyl, Oxazolyl, Isoxazolyl, Oxadiazolyl, Tetrazolyl, Pyridyl, Pyrimidinyl, Pyrazinyl, Pyradizinyl, Triazinyl, Tetrazinyl und dergleichen. Heterocyclische Gruppen gleichen Typs mit mindestens einem Stickstoffheteroatom können von den Resten R₅ und R₆ in obiger Formel (I) zusammen mit dem Stickstoffatom, an das sie gebunden sind, gebildet werden.

Die in den erfindungsgemäß hergestellten Verbindungen enthaltenen geradkettigen oder verzweigtkettigen Alkylenreste umfassen geradkettige C₁-C₁₀-Alkylenreste, wie z.B. Ethylen, Propylen, Butylen, Pentylen und Hexylen sowie verzweigte C₁-C₁₀-Alkylenreste, wie z.B. 1,1-Dimethylethylen, 1,3-Dimethylpropylen, 1-Methyl-3-ethylpropylen, 2,3-Dimethylbutylen, 1,3-Dimethylbutylen, 1,1-Dimethylbutylen, 1,2-Dimethylpentylen und 1,3-Dimethylhexylen.

Beispiele für erfindungsgemäß geeignete Substituenten sind C₁-C₆-Alkyl, Amino-C₁-C₆-alkyl, Hydroxy-C₁-C₆-alkenyl, C₁-C₆-Alkyloxy, C₂-C₆-Alkenyl, C₁-C₆-Alkanoyl, wie z.B. Acetyl und Propionyl, Nitro und Amino.

Die erfindungsgemäß hergestellten Polyalkenamine der Formel (I) sind verwendbar als Additive für flüssige oder pastöse Schmierstoffzusammensetzungen. Darin ist wenigstens eines der erfindungsgemäßen Polyalkenamine, gegebenenfalls in Kombination mit weiteren üblichen Schmierstoffzusätzen, enthalten. Beispiele für übliche Zusätze sind Korrosionsinhibitoren, Verschleißschutzadditive, Viskositätsverbesserer, Detergenzien, Antioxidantien, Antischaummittel, Schmierfähigkeitsverbesserer und Stockpunktverbesserer. Die erfindungsgemäßen Verbindungen sind üblicherweise in Mengen von etwa 1 bis 15 Gew.-%, vorzugsweise etwa 0,5 bis 10 Gew.-%, insbesondere 1 bis 5 Gew.-%, bezogen auf das Gesamtgewicht der Zusammensetzung, enthalten.

Beispiele für solche Schmierstoffe umfassen Öle und Fette für Kraftfahrzeuge und industriell eingesetzte Antriebsaggregate, wie insbesondere Motorenöle, Getriebeöle und Turbinenöle.

Die erfindungsgemäß hergestellten Verbindungen können außerdem in Kraftstoffzusammensetzungen, wie z.B. in Kraftstoffen für Ottound Dieselmotoren, als Additiv enthalten sein. Die erfindungsgemäßen Verbindungen dienen darin insbesondere als Detergenzien zur Reinhaltung des Kraftstoffeinlaßsystems. Aufgrund ihrer dispergierenden Eigenschaften üben sie einen positiven Einfluß auf den Motorschmierstoff aus, in welchen sie während des Betriebs gelangen können. Die erfindungsgemäß hergestellten Polyalkenamine werden handelsüblichen Kraftstoffen in Konzentrationen von etwa 20 bis 5000 mg/kg, vorzugsweise etwa 50 bis 1000 mg/kg Kraftstoff zudosiert. Die erfindungsgemäßen Additive können gegebenenfalls auch zusammen mit anderen bekannten Additiven zugesetzt werden.

Während in Schmierstoffzusammensetzungen vorzugsweise solche erfindungsgemäßen Additive verwendet werden, die ein zahlenmittleres Molekulargewicht von etwa 2000 bis 40000 aufweisen, eignen sich für die Verwendung als Kraftstoffadditiv insbesondere Verbindungen mit einem zahlenmittleren Molekulargewicht von etwa 150 bis 5000, vorzugsweise von etwa 500 bis 2500 und insbesondere von etwa 800 bis 1500.

Schließlich können erfindungsgemäß hergestellte Verbindungen auch in Kombination mit anderen Additiven, insbesondere Detergenzien und Dispergatoren, enthalten sein. Besonders bevorzugt ist eine Kombination mit beispielsweise aus der US 4,832,702 bekannten Polyisobutylaminen.

Die Prüfung der erfindungsgemäßen Produkte als Kraftstoffadditive, besonders auf ihre Eignung als Ventil- und Vergaserreiniger, geschieht mit Hilfe von Motortests, die in Prüfstandversuchen mit einem 1,2 l Opel Kadett-Motor gemäß CEC-F-04-A-87 durchgeführt werden.

Zur Prüfung der erfindungsgemäßen Produkte hinsichtlich ihrer Dispergatoreigenschaften kann ein "Tüpfeltest", wie er z.B. von A. Schilling in "Les Huiles pour Moteurs et la Graissage des Moteur", Vol. 1, 1962, S. 89f, in etwas modifizierter Form beschrieben ist, herangezogen werden.

Die vorliegende Erfindung wird anhand folgender Ausführungsbeispiele näher erläutert.

### Ausführungsbeispiele

Als Einsatzmaterial wurde in den folgenden Beispielen eine 50 %ige Lösung von Polyisobutenepoxid in Mihagol verwendet, das durch Epoxidation von Glissopal®1000 (Handelsprodukt von BASF AG) hergestellt wurde. Zur Charakterisierung der Aminoalkane bzw. der entsprechenden Aminoalkohole wurden Aminzahlen und Hydroxylzahlen bestimmt.

Der in den folgenden Beispielen verwendete Katalysator mit Dehydratisierungs- und Hydrierungseigenschaften wurde gemäß EP 0 696 572 hergestellt und hatte folgende Zusammensetzung (Angaben jeweils bezogen auf das Gesamtgewicht der katalytisch aktiven Masse):
30 Gew.-% ZrO₂
50 Gew.-% NiO
18 Gew.-% CuO
1,5 Gew.-% MoO₃
0,5 Gew.-% Na₂O

### Beispiel 1: Einstufige, kontinuierliche Umsetzung mit Ammoniak

In einem 1 l Rohrreaktor, der mit 500 g Katalysator befüllt ist, werden 125 ml/Stunde einer 50 %igen Lösung von Polyisobutenepoxid in Mihagol mit 250 ml/Stunde Ammoniak kontinuierlich umgesetzt. Die Reaktionstemperatur liegt im Reaktor zwischen 200 und 205°C. Der Druck beträgt 250 bar und die Wasserstoffmenge 100 l/Stunde. Im Vakuum werden die leichtflüchtigen Bestandteile (Wasser, Ammoniak und Mihagol) abdestilliert (bis 70°C Sumpftemperatur bei 3 mbar). Die Aminzahl des erhaltenen Produkts liegt bei 30,0 und die Hydroxylzahl bei 2,0.

### Beispiel 2: Einstufige, diskontinuierliche Umsetzung mit Ammoniak

225 g Polyisobutenepoxid, gelöst in 225 g Mihagol und 5 g Wasser, werden mit 100 g Katalysator versetzt. Im Autoklaven wird nach Zugabe von 450 ml Ammoniak bei einem Wasserstoffdruck von 200 bar 4 Stunden auf 200°C erhitzt. Nach Abtrennung sämtlicher Leichtsieder im Vakuum erhält man ein lösungsmittelfreies Produkt mit einer Aminzahl von 29,2 und einer Hydroxylzahl von 4, d.h. der Aminoalkohol wurde dehydratisiert und hydriert.

### Beispiel 3: Zweistufige, diskontinuierliche Umsetzung mit Ammoniak

200 g Polyisobutenepoxid werden in einem Gemisch aus 200 g Mihagol, 300 ml THF und 12 g Wasser gelöst. Im Autoklaven wird nach Zugabe von 300 ml Ammoniak bei einem Stickstoffdruck von 200 bar 12 Stunden auf 200°C erhitzt. Im Vakuum werden die leichtflüchtigen Bestandteile (Wasser, THF, Mihagol) abdestilliert. Die Aminzahl des Produkts liegt bei 32,8 und die Hydroxylzahl bei 32,2, d.h. es liegt der gewünschte Aminoalkohol vor.

100 g des Aminoalkohols werden in 400 g Mihagol gelöst und mit 100 g Katalysator versetzt. Im Autoklaven wird nach Zugabe von 500 ml Ammoniak bei einem Wasserstoffdruck von 200 bar 24 Stunden auf 200°C erhitzt. Nach Abtrennung sämtlicher Leichtsieder im Vakuum erhält man ein lösungsmittelfreies Produkt mit einer Aminzahl von 29 und einer Hydroxylzahl von 2, d.h. der Aminoalkohol wurde dehydratisiert und hydriert.

## Patentansprüche

1. Verfahren zur Herstellung von Polyalkenaminen der Formel (I) 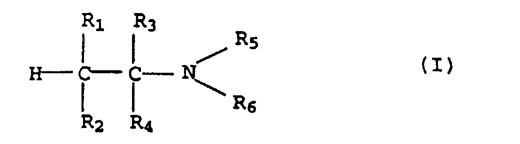 worin
R₁, R₂, R₃ und R₄ unabhängig voneinander für ein Wasserstoffatom oder einen gegebenenfalls substituierten, gesättigten oder ein- oder mehrfach ungesättigten aliphatischen Rest mit einem zahlenmittleren Molekulargewicht von bis zu 40000 steht; wobei wenigstens einer der Reste R₁ bis R₄ ein zahlenmittleres Molekulargewicht von 150 bis 40000 aufweist; und
R₅ und R₆ unabhängig voneinander für ein Wasserstoffatom,einen Alkyl-, Cycloalkyl-, Hydroxyalkyl-, Aminoalkyl-, Alkenyl-, Alkinyl-, Aryl-, Arylalkyl-, Alkylaryl-, Heteroarylrest oder einen Alkyleniminrest der Formel (II) 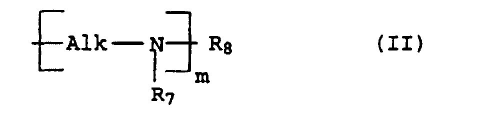 stehen, worin
Alk für einen geradkettigen oder verzweigten Alkylenrest steht;
m für einen ganzzahligen Wert von 0 bis 10 steht; und
R₇ und R₈ unabhängig voneinander für ein Wasserstoffatom, einen Alkyl-, Cycloalkyl-, Hydroxyalkyl-, Aminoalkyl-, Alkenyl-, Alkinyl-, Aryl-, Arylalkyl-, Alkylaryl- oder Heteroarylrest stehen oder zusammen mit dem Stickstoffatom, an das sie gebunden sind, einen Heterozyklus bilden;
oder R₅ und R₆ zusammen mit dem Stickstoffatom, an das sie gebunden sind, für einen Heterozyklus stehen; wobei jeder der Reste R₅, R₆, R₇ und R₈ durch weitere Hydroxy- oder Aminogruppen tragende Alkylreste substituiert sein kann;
**dadurch gekennzeichnet, daß** man
ein Epoxid der Formel (IV) 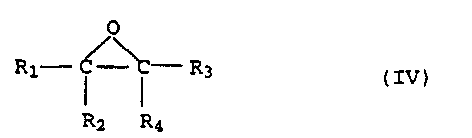 worin R₁, R₂, R₃ und R₄ die oben angegebenen Bedeutungen besitzen mit einer Stickstoffverbindung der Formel (V) 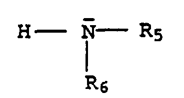 worin R₅ und R₆ die oben angegebenen Bedeutungen besitzen, zum Aminoalkohol der Formel (VI) 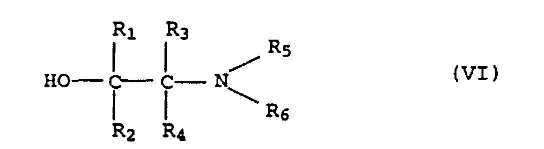 umsetzt;
den Aminoalkohol der Formel (VI) katalytisch dehydratisiert,
und das gebildete Olefin zum Amin der Formel (I) hydriert.

2. Verfahren nach Anspruch 1, **dadurch gekennzeichnet, daß** man das Epoxid der Formel (IV) mit der Stickstoffverbindung der Formel (V) in Gegenwart von Wasserstoff und von einem Katalysator einstufig umsetzt, welcher Dehydratisierungs- und zugleich Hydrierungseigenschaften besitzt.

3. Verfahren nach Anspruch 1, **dadurch gekennzeichnet, daß** man das Epoxid der Formel (IV) zuerst mit der Stickstoffverbindung der Formel (V) in Gegenwart eines Alkoxylierungskatalysators zum Aminoalkohol der Formel (VI) umsetzt und gegebenenfalls nicht umgesetzte Reaktanden abtrennt; und anschliessend den Aminoalkohol (VI) in Gegenwart eines Katalysators, welcher Dehydratisierungs- und zugleich Hydrierungseigenschaften besitzt, hydriert.

4. Verfahren nach einem der Ansprüche 2 oder 3, **dadurch gekennzeichnet, daß** der Katalysator mit Dehydratisierungs- und Hydrierungseigenschaften ausgewählt ist unter Zeolithen oder porösen Oxiden von Al, Si, Ti, Zr, Nb, Mg oder Zn, sauren Ionenaustauschern und Heteropolysäuren, welche jeweils mindestens ein Hydriermetall tragen.

5. Verfahren nach Anspruch 4, **dadurch gekennzeichnet, daß** das Hydriermetall ausgewählt ist unter Ni, Co, Cu, Fe, Pd, Pt, Ru, Rh oder Kombinationen davon.

6. Verfahren nach Anspruch 5, **dadurch gekennzeichnet, daß** der Katalysator (katalytisch aktive Masse) 30 Gew.-% einer Zirkoniumverbindung, berechnet als ZrO₂, 50 Gew.-% einer Nickelverbindung, berechnet als NiO, 18 Gew.-% einer Kupferverbindung, berechnet als CuO, 1,5 Gew.-% eine Molybdänverbindung, berechnet als MoO₃ und 0,5 Gew.-% einer Natriumverbindung, berechnet als Na₂O, enthält.

7. Verfahren nach einem der vorhergehenden Ansprüche, **dadurch gekennzeichnet, daß** man Stickstoffverbindung und Epoxid in einem molaren Verhältnis von 1:1 bis 40:1 einsetzt.

8. Verfahren nach einem der vorhergehenden Ansprüche, **dadurch. gekennzeichnet, daß** die Reaktionstemperatur 80 bis 250°C beträgt.

9. Verfahren nach einem der vorhergehenden Ansprüche, **dadurch gekennzeichnet, daß** ein Wasserstoffdruck von bis zu 600 bar eingestellt wird.

10. Verfahren nach einem der vorhergehenden Ansprüche, **dadurch gekennzeichnet, daß** man ein Epoxid der Formel (IV) einsetzt, worin einer der Reste R₁ bis R₄ ein zahlenmittleres Molekulargewicht von 150 bis 40000 aufweist.

11. Verfahren nach Anspruch 10, **dadurch gekennzeichnet, daß** das Epoxid von einem Polyalken abgeleitet ist, das ein Homo- oder Copolymer von C₂- bis C₃₀-Alkenen ist.

12. Verfahren nach Anspruch 11, **dadurch gekennzeichnet, daß** das Polyalken abgeleitet ist von wenigstens einem 1-Alken, ausgewählt unter Ethylen, Propylen, 1-Buten und Isobuten.

13. Verfahren nach einem der vorhergehenden Ansprüche, **dadurch gekennzeichnet, daß** die Stickstoffverbindung der Formel (V) ausgewählt ist unter NH₃, Mono- und Dialkylaminen und Alkylendiaminen mit wenigstens einer primären oder sekundären Aminogruppe.

14. Reaktionsprodukt, erhältlich durch
a) Epoxidierung eines reaktiven Polyalkens der Formel (III) 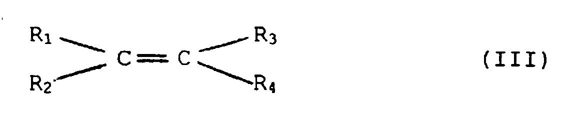 worin R₁ bis R₄ die oben angegebenen Bedeutungen besitzen, zu einem Epoxid obiger Formel (IV)
b) Umsetzung des Epoxids der Formel (IV) mit einer Stickstoffverbindung obiger Formel (V) zu einem Aminoalkohol obiger Formel (VI); und
c) katalytische Dehydratisierung des Aminoalkohols der Formel (VI) und Hydrierung des dehydratisierten Produkts.

15. Reaktionsprodukt nach Anspruch 14, welches abgeleitet ist von einem Polyalken der allgemeinen Formel (III), welches aus C₂-C₄-Alken-Monomeren aufgebaut ist.

16. Reaktionsprodukt nach Anspruch 15, wobei das C₂-C₄-Alken 1-Buten oder Isobuten ist.

17. Reaktionsprodukt nach Anspruch 14, abgeleitet von reaktivem Polyalken mit hohem Anteil an endständigen Doppelbindungen.

18. Reaktionsprodukt nach Anspruch 14, wobei die Stickstoffverbindung der Formel (V) ausgewählt ist unter Ammoniak, Ethylen-1,2-diamin, Propylen-1,2-diamin, Propylen-1,3-diamin, Butylendiaminen, den Mono-, Di- und Trialkylderivaten dieser Amine, Polyalkylenpolyaminen, deren Alkylenreste nicht mehr als 6 C-Atome besitzen, und N-Amino-C₁-C₆-alkylpiperazin.

19. Reaktionsprodukt nach Anspruch 18, welches abgeleitet ist von einem Polyalken der allgemeinen Formel (III), welches aus 1-Buten- oder Isobuten-Monomeren aufgebaut ist und Ammoniak.

20. Kraftstoffzusammensetzung, enthaltend als Additiv zur Reinhaltung des Kraftstoffeinlaßsystems wenigstens ein Reaktionsprodukt nach einem der Ansprüche 14 bis 19 in einer Konzentration von etwa 20 bis 5000 mg/kg Kraftstoff.

21. Schmierstoffzusammensetzung, enthaltend als Additiv wenigstens ein Reaktionsprodukt nach einem der Ansprüche 14 bis 19 in einem Anteil von etwa 1 bis 15 Gew.-% bezogen auf das Gesamtgewicht der Zusammensetzung.

22. Additivzusammensetzung, umfassend ein Reaktionsprodukt nach einem der Ansprüche 14 bis 19.

23. Verwendung eines Reaktionsprodukts nach einem der Ansprüche 14 bis 19 als Detergensadditiv für Kraftstoffzusammensetzungen.

## Claims

1. A process for the preparation of polyalkeneamines of the formula (I) 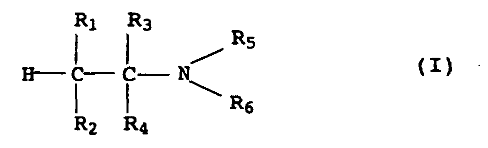 where
R₁, R₂, R₃ and R₄ , independently of one another, are each hydrogen or an unsubstituted or substituted, saturated or mono- or polyunsaturated aliphatic radical having a number-average molecular weight of up to 40000, at least one of the radicals R₁ to R₄ having a number average molecular weight of from 150 to 40000, and
R₅ and R₆, independently of one another, are each hydrogen, alkyl, cycloalkyl, hydroxyalkyl, aminoalkyl, alkenyl, alkynyl, aryl, arylalkyl, alkylaryl, hetaryl or an alkyleneimine radical of the formula (II) 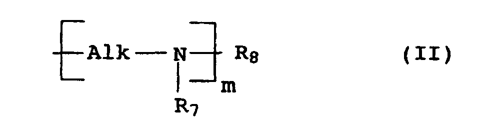 where
Alk is straight-chain or branched alkylene,
m is an integer from 0 to 10, and
R₇ and R₈, independently of one another, are each hydrogen, alkyl, cycloalkyl, hydroxyalkyl, aminoalkyl, alkenyl, alkynyl, aryl, arylalkyl, alkylaryl or hetaryl or, together with the nitrogen atom to which they are bonded, form a heterocyclic structure,
or R₅ and R₆, together with the nitrogen atom to which they are bonded, form a heterocyclic structure, it being possible for each of the radicals R₅, R₆, R₇ and R₈ to be substituted by further alkyl radicals carrying hydroxyl or amino groups,
wherein
an epoxide of the formula (IV) 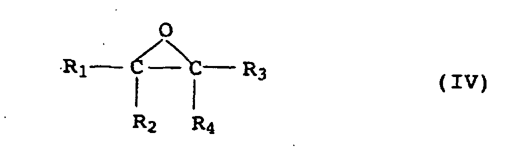 where R₁, R₂, R₃ and R₄ have the abovementioned meanings, is reacted with a nitrogen compound of the formula (V) 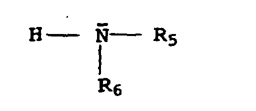 where R₅ and R₆ have the abovementioned meanings, to give the amino alcohol of the formula (VI) 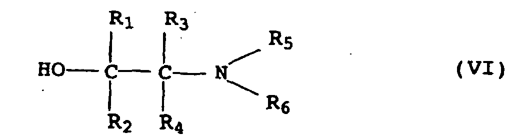 the amino alcohol of the formula (VI) is catalytically dehydrated and the olefin formed is hydrogenated to give the amine of the formula (I).

2. A process as claimed in claim 1, wherein the epoxide of the formula (IV) is reacted in one stage with the nitrogen compound of the formula (V) in the presence of hydrogen and of a catalyst which has dehydrating and at the same time hydrogenating properties.

3. A process as claimed in claim 1, wherein the epoxide of the formula (IV) is first reacted with the nitrogen compound of the formula (V) in the presence of an alkoxylation catalyst to give the amino alcohol of the formula (VI) and, if required, unconverted reactants are separated off, and the amino alcohol (VI) is then hydrogenated in the presence of a catalyst which has dehydrating and at the same time hydrogenating properties.

4. A process as claimed in claim 2 or 3, wherein the catalyst having dehydrating and hydrogenating properties is selected from zeolites or porous oxides of Al, Si, Ti, Zr, Nb, Mg or Zn, acidic ion exchangers and heteropolyacids, each of which carries at least one hydrogenation metal.

5. A process as claimed in claim 4, wherein the hydrogenation metal is selected from Ni, Co, Cu, Fe, Pd, Pt, Ru, Rh and combinations thereof.

6. A process as claimed in claim 5, wherein the catalyst (catalytically active material) contains 30 % by weight, calculated as ZrO₂, of a zirconium compound, 50 % by weight, calculated as NiO, of a nickel compound 18 % by weight, calculated as CuO, of a copper compound, 1.5 % by weight, calculated as MoO₃, of a molybdenum compound and 0.5 % by weight, calculated as Na₂O, of a sodium compound.

7. A process as claimed in any of the preceding claims, wherein the nitrogen compound and epoxide are used in a molar ratio of from 1:1 to 40:1.

8. A process as claimed in any of the preceding claims, wherein the reaction temperature is from 80 to 250°C.

9. A process as claimed in any of the preceding claims, wherein a hydrogen pressure of up to 600 bar is established.

10. A process as claimed in any of the preceding claims, wherein an epoxide of the formula (IV), where one of the radicals R₁ to R₄ has a number average molecular weight of from 150 to 40000, is used.

11. A process as claimed in claim 10, wherein the epoxide is derived from a polyalkene which is a homo- or copolymer of C₂-C₃₀-alkenes.

12. A process as claimed in claim 11, wherein the polyalkene is derived from at least one 1-alkene, selected from ethylene, propylene, 1-butene and isobutene.

13. A process as claimed in any of the preceding claims, wherein the nitrogen compound of the formula (V) is selected from NH₃, monoalkylamines, dialkylamines and alkylenediamines having at least one primary or secondary amino group.

14. A reaction product obtainable by
a) epoxidation of a reactive polyalkene of the formula (III) 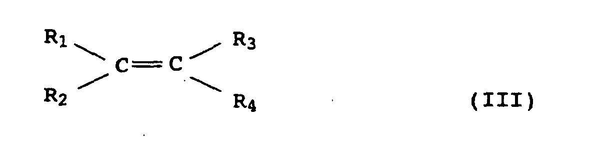 where R₁ to R₄ are each as defined above,
to form an epoxide of the above formula (IV)
b) reaction of the epoxide of the formula (IV) with a nitrogen compound of the above formula (V) to form an amino alcohol of the above formula (VI); and
c) catalytic dehydration of the amino alcohol of formula (VI) and hydrogenation of the dehydrated product.

15. A reaction product as claimed in claim 14 and derived from a general formula (III) polyalkene which has been constructed from C₂-C₄-alkene monomers.

16. A reaction product as claimed in claim 15, wherein the C₂-C₄-alkene is 1-butene or isobutene.

17. A reaction product as claimed in claim 14 and derived from a reactive polyalkene having a high fraction of terminal double bonds.

18. A reaction product as claimed in claim 14, wherein the nitrogen compound of the formula (V) is selected from the group consisting of ammonia, ethylene-1,2-diamine, propylene-1,2-diamine, propylene-1,3-diamine, butylenediamines, the mono-, di- and trialkyl derivatives of these amines, polyalkylenepolyamines whose alkylene radicals have not more than 6 carbon atoms and N-amino-C₁-C₆-alkyl-piperazine.

19. A reaction product as claimed in claim 18 and derived from a general formula (III) polyalkene which has been constructed from 1-butene or isobutene monomers and ammonia.

20. A motor fuel composition containing at least one reaction product as claimed in any of claims 14 to 19 in a concentration of about 20-5 000 mg/kg of fuel as an additive for keeping the fuel intake system clean.

21. A lubricant composition containing at least one reaction product as claimed in any of claims 14 to 19 in a fraction of about 1-15% by weight based on the total weight of the composition as an additive.

22. An additive composition comprising a reaction product as claimed in any of claims 14 to 19.

23. The use of a reaction product as claimed in any of claims 14 to 19 as a detergent additive for motor fuel compositions.

## Revendications

1. Procédé de préparation de polyalcénamides de formule (I) 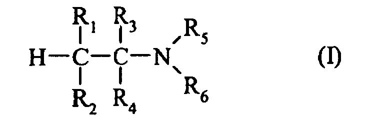 dans laquelle
R₁, R₂, R₃ et R₄ représentent chacun indépendamment des autres un atome d'hydrogène ou un radical aliphatique éventuellement substitué, saturé ou une ou deux fois insaturé, ayant une masse moléculaire moyenne en nombre allant jusqu'à 40 000 ; au moins l'un des radicaux R₁ à R₄ ayant une masse moléculaire moyenne en nombre de 150 à 40 000 ; et
R₅ et R₆ représentent chacun indépendamment de l'autre un atome d'hydrogène, un radical alkyle, cycloalkyle, hydroxyalkyle, aminoalkyle, alcényle, alcynyle, aryle, arylalkyle, alkylaryle, hétéroaryle, ou un radical alcylénimine de formule (II) 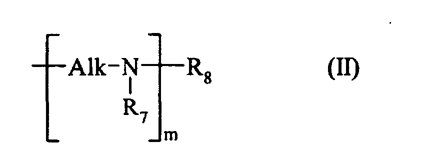 dans laquelle
Alk désigne un radical alkylène à chaîne droite ou ramifiée ;
m est un nombre entier de 0 à 10 ; et
R₇ et R₈ représentent chacun indépendamment de l'autre un atome d'hydrogène, un radical alkyle, cycloalkyle, hydroxyalkyle, aminoalkyle, alcényle, alcynyle, aryle, arylalkyle, alkylaryle ou hétéroaryle, ou avec l'atome d'azote auquel ils sont liés forment un hétérocycle ;
ou encore R₅ et R₆, avec l'atome d'azote auquel ils sont liés, représentent un hétérocycle ; chacun des radicaux R₅, R₆, R₇ et R₈ pouvant être substitué par d'autres radicaux alkyle portant des groupes hydroxy ou amino ;
**caractérisé en ce que** :
on fait réagir un époxyde de formule (IV) 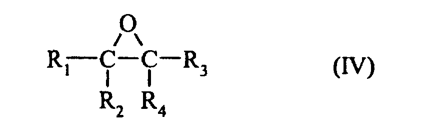
dans laquelle R₁, R₂, R₃ et R₄ ont les significations données ci-dessus, avec un composé azoté de formule (V) 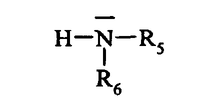 dans laquelle R₅ et R₆ ont les significations données ci-dessus, pour obtenir un amino-alcool de formule (VI) 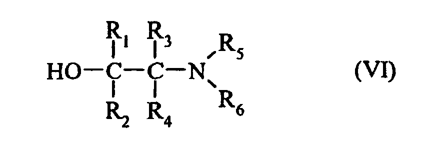 on soumet l'aminoalcool de formule (VI) à une déshydratation catalytique, et on hydrogène en l'amine de formule (I) l'oléfine ainsi formée.

2. Procédé selon la revendication 1, **caractérisé en ce qu'**on fait réagir en une étape l'époxyde de formule (IV) avec le composé azoté de formule (V) en présence d'hydrogène et d'un catalyseur, lequel présente des propriétés de déshydratation et simultanément d'hydrogénation.

3. Procédé selon la revendication 1, **caractérisé en ce qu'**on fait d'abord réagir l'époxyde de formule (IV) avec le composé azoté de formule (V) en présence d'un catalyseur d'alcoxylation pour obtenir l'aminoalcool de formule (VI), et éventuellement on le sépare des matières de départ n'ayant pas réagi ; puis on hydrogène l'aminoalcool (VI) en présence d'un catalyseur qui présente des propriétés de déshydratation et simultanément d'hydrogénation.

4. Procédé selon l'une des revendications 2 ou 3, **caractérisé en ce que** le catalyseur présentant des propriétés de déshydratation et d'hydrogénation est choisi parmi les zéolites ou les oxydes poreux d'Al, de Si, de Ti, de Zr, de Nb, de Mg ou de Zn, les échangeurs d'ions acides et les hétéropolyacides, qui chacun porte au moins un métal d'hydrogénation.

5. Procédé selon la revendication 4, **caractérisé en ce que** le métal d'hydrogénation est choisi parmi Ni, Co, Cu, Fe, Pd, Pt, Ru, Rh ou les combinaison de ceux-ci.

6. Procédé selon la revendication 5, **caractérisé en ce que** le catalyseur (masse catalytiquement active) contient 30 % en poids d'un composé du zirconium, calculés en ZrO₂, 50 % en poids d'un composé du nickel, calculés en NiO, 18 % en poids d'un composé du cuivre, calculés en CuO, 1,5 % en poids d'un composé du molybdène, calculés en MoO₃, et 0,5 % en poids d'un composé du sodium, calculés en Na₂O.

7. Procédé selon l'une des revendications précédentes, **caractérisé en ce qu'**on utilise le composé azoté et l'époxyde selon un rapport en moles de 1:1 à 40:1.

8. Procédé selon l'une des revendications précédentes, **caractérisé en ce que** la température de la réaction est de 80 à 250°C.

9. Procédé selon l'une des revendications précédentes, **caractérisé en ce qu'**on ajuste une pression d'hydrogène allant jusqu'à 600 bar.

10. Procédé selon l'une des revendications précédentes, **caractérisé en ce qu'**on utilise un époxyde de formule (IV) dans laquelle l'un des radicaux R₁ à R₄ a une masse moléculaire moyenne en nombre de 150 à 40 000.

11. Procédé selon la revendication 10, **caractérisé en ce que** l'époxyde dérive d'un polyalcène qui est un homo- ou un copolymère d'alcènes en C₂ à C₃₀.

12. Procédé selon la revendication 11, **caractérisé en ce que** le polyalcène dérive d'au moins un 1-alcène, choisi parmi l'éthylène, le propylène, le 1-butène et l'isobutène.

13. Procédé selon l'une des revendications précédentes, **caractérisé en ce que** le composé azoté de formule (V) est choisi parmi NH₃, les mono- et les dialkylamines, et les alkylènediamines ayant au moins un groupe amino primaire ou secondaire.

14. Produit de réaction pouvant être obtenu par
a) époxydation d'un polyalcène réactif de formule (III) 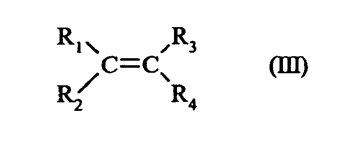 dans laquelle R₁ à R₄ ont les significations données ci-dessus, pour donner un époxyde ayant la formule (IV) ci-dessus,
b) réaction de l'époxyde de formule (IV) avec un composé azoté ayant la formule (V) ci-dessus pour donner un amino-alcool ayant la formule (VI) ci-dessus ; et
c) déshydratation catalytique de l'aminoalcool de formule (VI) et hydrogénation du produit déshydraté.

15. Produit de réaction selon la revendication 14, qui dérive d'un polyalcène de formule générale (III), qui est constitué d'alcènes monomères en C₂-C₄.

16. Produit de réaction selon la revendication 15, dans lequel l'alcène en C₂-C₄ est le 1-butène ou l'isobutène.

17. Produit de réaction selon la revendication 14, qui dérive d'un polyalcène réactif ayant une proportion élevée de doubles liaisons terminales.

18. Produit de réaction selon la revendication 14, dans lequel le composé azoté de formule (V) est choisi parmi l'ammoniac, l'éthylène-1,2-diamine, la propylène-1,2-diamine, la propylène-1,3-diamine, les butylènediamines, les dérivés mono-, di- et trialkylés de ces amines, les polyalkylènepolyamines, dont les radicaux alkylènes n'ont pas plus de 6 atomes de carbone, et les N-amino-(alkyle en C₁-C₆)pipérazines.

19. Produit de réaction selon la revendication 18, qui dérive d'un polyalcène de formule générale (III), qui est constitué de 1-butènes ou d'isobutènes monomères, et d'ammoniac.

20. Composition de carburant, contenant en tant qu'additif pour maintenir propre le système d'admission de carburant au moins un produit de réaction selon l'une des revendications 14 à 19 à une concentration d'environ 20 à 5000 mg/kg de carburant.

21. Composition lubrifiante contenant en tant qu'additif au moins un produit de réaction selon l'une des revendications 14 à 19 en une quantité d'environ 1 à 15 % en poids par rapport au poids total de la composition.

22. Composition additive, comprenant un produit de réaction selon l'une des revendications 14 à 19.

23. Utilisation d'un produit de réaction selon l'une des revendications 14 à 19 en tant qu'additif détergent pour compositions de carburants.
